# EUROPÄISCHE PATENTSCHRIFT

(11) **EP 1 973 581 B1**
(45) Veröffentlichungstag und Bekanntmachung des Hinweises auf die Patenterteilung: **14.07.2010**
(21) Anmeldenummer: 06841160.2
(22) Anmeldetag: 22.12.2006
(51) Int. Cl.: A61L 15/20, A61L 15/28, A61L 15/46

(54) **ANTISEPTISCHE ALGINATZUBEREITUNG**
ANTISEPTIC ALGINATE PREPARATION
PRÉPARATION D'ALGINATES ANTISEPTIQUE

(30) Priorität: 16.01.2006 DE 102006001954
(43) Veröffentlichungstag der Anmeldung: 01.10.2008
(73) Patentinhaber: Lohmann & Rauscher GmbH & Co. KG, 56567 Neuwied (DE)
(72) Erfinder: ROHRER, Christian, A-4020 Linz (AT); LEUPRECHT, Helmut, A-1130 Wien (AT); ALUPEI, Iulian-Corneliu, 56564 Neuwied (DE)
(74) Vertreter: Flaccus, Rolf-Dieter
(86) Internationale Anmeldenummer: PCT/EP2006/012525
(87) Internationale Veröffentlichungsnummer: WO 2007/087888

(56) Entgegenhaltungen:
- EP-A- 0 532 275
- EP-A- 0 613 692
- WO-A-94/17227
- WO-A-96/17595
- US-A- 5 690 955
- US-A- 5 693 624

## Beschreibung

Die vorliegende Erfindung betrifft Zubereitungen, die zur Herstellung von Wundauflagen oder Verbandmaterialien geeignet sind und mindestens ein Alginat sowie mindestens eine antiseptisch wirksame Substanz umfassen.

Ferner betrifft die Erfindung Verfahren zur Herstellung von antiseptisch wirksamen Alginatzubereitungen und deren Verwendung.

Die Salze der Alginsäure werden als Alginate bezeichnet. Alginsäure ist ein farbloses, Carboxy-Gruppen enthaltendes, zu den Polyuronsäuren gehörendes Polysaccharid aus 1,4-glykosidisch verknüpften D-Mannuronsäure-Einheiten mit gelegentlichen Einschüben von L-Guluronsäure. Alginsäure kann bis zu 40 Gew.-% der Trockenmasse von Braunalgen ausmachen. Die Alkalisalze der Alginsäure, das Ammoniumsalz der Alginsäure sowie das Magnesiumsalz der Alginsäure sind wasserlöslich. Insbesondere das Natrium-Alginat, das auch als Algin bezeichnet wird, hat eine große Bedeutung als Verdickungsmittel, Emulgator bzw. Emulsionsstabilisator und Gelgrundlage für die Nahrungsmittel-, Pharma- und Kosmetikindustrie. Calcium-Alginate und Zink-Alginate sind dagegen wasserunlöslich, ebenso wie Alginsäure. Durch Zusatz von wasserunlöslichem Calcium-Alginat zu Natrium-Alginatgelen läßt sich deren Viskosität erhöhen.

Die Verwendung von Calcium-Alginat zur Herstellung von Alginatfasern sowie aus Alginatfasern hergestellte Wundauflagen sind bekannt. Alginatfasern werden hergestellt, indem die Alginsäure aus den Algen mit Hilfe von Sodalösung extrahiert wird. Die resultierende Natrium-Alginatlösung wird gereinigt und in ein Fällbad mit schwach saurer CaCl₂-Lösung gepreßt.

EP 0 586 260 A1 beschreibt Alginat-Gele in Gestalt einer faserigen Paste, die einen Alginat-Gehalt von 2 bis 11 Gew.-% aufweist und durch Behandlung von wasserunlöslichen oder in Wasser quellbaren Alginatfasern mit einer wäßrigen Lösung eines solubilisierenden Salzes hergestellt wird.

US Patent 5,470,576 offenbart ein Verfahren zur Herstellung von Alginat-haltigen Wundauflagen, bei dem ein weiches, absorptionsfähiges Gewebe mit einem Alginat imprägniert wird, indem das Gewebe in eine wäßrige Natrium-Alginatlösung getaucht wird, der anschließend Calciumchlorid zugesetzt wird, um Calcium-Alginat auszufällen. Diese Wundauflagen sollen bei Kontakt mit einer Wunde haemostatisch wirken.

In EP 0 783 605 B1 werden Wundverbände beschrieben, die Alginatfasern enthalten und durch gemeinsames Verspannen und Festwerden eines Alginats und einer wasserlöslichen Carboxymethylcellulose hergestellt werden, wobei das Alginat eine vernetzte Form hat und das Alginat, aus dem die Fasern mit versponnen werden, einen G-Gehalt von mindestens 35 Gew.-% aufweist.

EP 1 435 247 A1 betrifft eine mehrschichtige Wundauflage, die ein Gewebe aus Alginatfasern und eine nicht mit der Wunde in Kontakt zu bringende Schicht umfaßt, welche einen Superabsorber enthält.

Wundauflagen oder Verbände aus natürlichen Alginatfasern weisen eine hohe Sekretaufnahmefähigkeit auf. Dadurch sind diese Wundauflagen auch zur Versorgung stark exsudierender Wunden, beispielsweise Ulcera, Decubiti und frische Spalthauteatnahmen, sowie zur Versorgung von infizierten Wunden geeignet. Ihre hohe Sekretaufnahme erlaubt Verbandwechsel in größeren Zeitabständen.

Besonders vorteilhaft ist, daß Wundauflagen aus Alginatfasern bei Kontakt mit Wundexsudat oder Blut ein Gel bilden. Dadurch kann sich die Wundauflage den Wundkonturen anpassen und auch im Exsudat enthaltene Bakterien im Gel binden. Die Wunde wird ständig feucht gehalten, Epithel kann sich gut entwickeln, und durch das optimale Mikroklima wird die Wundheilung beschleunigt.

Ein weiterer Vorteil von Wundauflagen oder Verbänden aus natürlichen Alginatfasern ist, daß sie nicht mit der Wunde verkleben. Daher wird das junge Gewebe beim Verbandwechsel nicht verletzt und der Heilungsvorgang wird nicht unterbrochen.

Mit Krankheitserregern verunreinigte bzw. infizierte Wunden müssen jedoch auch antiseptisch behandelt werden, weil
- sich eine Infektion entwickeln kann, solange die Wunde kolonisiert ist,
- der Wundheilungsprozeß nicht oder nur verzögert zum Abschluß kommt, solange die Wunde infiziert ist,
- sich die Wundinfektion ausbreiten und zu einer Sepsis führen kann, und
- bei einer Wundkolonisation mit multiresistenten Keimen eine Weiterverbreitung der Erreger zu verhindern ist.

Auch bei Verbrennungswunden besteht die Notwendigkeit einer frühen Prävention von Wundinfektionen, insbesondere bei zu erwartender Kontamination großer Wundflächen.

Es besteht daher ein Bedarf an Wundauflagen oder Verbänden für die Versorgung von Wunden, mit denen Wunden antiseptisch behandelt werden können.

Allerdings ist die Behandlung von akuten und chronischen Wunden mit Lokaltherapeutika, insbesondere mit Antiseptika, nach heutiger Auffassung nur bei besonderen Indikationen angezeigt, da viele der gängigen Antiseptika bei einer Bewertung ihres Kosten-Nützen-Risiko-Verhältnisses als unakzeptabel angesehen werden. Moderne Lokalantiseptika, die dagegen ein breites Wirkspektrum und eine gute Verträglichkeit aufweisen, sind beispielsweise Octenidin und Polyhexanid (= Polyhexamethylenbiguanid; PHMB). Eine kurzzeitige Anwendung von Octenidin wird besonders bei mikrobiell kontaminierten akuten Wunde befürwortet, wohingegen Polyhexanid aufgrund seiner vergleichsweise langsam eintretenden Wirkung eher für wiederholte Anwendungen auf chronisch schlecht heilenden oder empfindlichen Wunden empfohlen wird. Nachteilig bei der Verwendung von Polyhexanid ist jedoch, daß dieses Antiseptikum seine Wirksamkeit in Gegenwart bereits geringer Mengen negativ geladener Ionen verliert.

WO 02/36866 A1 offenbart Polysaccharid-Fasern mit wasserabsorbierenden Eigenschaften, vorzugsweise aus Alginat oder aus der Kombination von Alginat mit einem anderen Polysaccharid-Material, beispielsweise absorptionsverbessernde Carboxymethylcellulose, die eine Silberverbindung als antimikrobiell wirksames Mittel enthalten. In WO 02/36866 A1 werden auch Wundauflagen beschrieben, die aus diesen Polysaccharid-Fasern hergestellt werden.

WO 03/022317 A1 beschreibt eine antibakterielle Wundauflage auf Basis von gelbildenden Fasern wie Carboxymethylcellulose oder Alginatfasern, an die Silberionen gleichmäßig über einen Teil der zur Verfügung stehenden reversiblen Bindungsstellen für Kationen gebunden sind.

Die Verwendung von Silber und Silberverbindungen in der Wundbehandlung gilt wegen der kurzen Stabilität der gebräuchlichen Zubereitungen, der möglichen Resorption von Silberionen und der Oberflächenzerstörung der Haut durch Eiweißfällung als überholt.

Der vorliegenden Erfindung lag daher die Aufgabe zugrunde, eine Wundauflage bzw. einen Verband für die antiseptische Behandlung von Wunden bereitzustellen, die eine gute antimikrobielle Aktivität aufweisen, mit denen das Prinzip der feuchten Wundbehandlung fortgeführt werden kann, die die wundheilung nicht nachteilig beeinflussen und die gut verträglich sind, so daß die gegenüber den seit langem etablierten Präparate bestehenden Bedenken auf diese Wundauflagen nicht zutreffen.

Diese Aufgabe wird überraschenderweise durch eine Zubereitung gelöst, die mindestens ein Alginat und mindestens eine antiseptisch wirksame Substanz umfaßt, die aus der Gruppe ausgewählt ist, die Biguanid-Derivate, Octenidin und Taurolidin umfaßt.

Gegenstand der vorliegenden Erfindung ist somit eine Zubereitung auf Basis von Alginatfasern, die mindestens ein Antiseptikum umfaßt, das aus der Gruppe ausgewählt ist, die Biguanid-Derivate, Octenidin und Taurolidin umfaßt.

Bei den Alginatfasern handelt es sich vorzugsweise um Calcium-Alginatfasern, aber auch Zink-Alginatfasern sind geeignet. Die Zubereitungen, die Alginatfasern umfassen können in Form von losen Fasern oder in Form eines Faserverbundes, vorzugsweise eines losen Faserverbunden, beispielsweise ein Kardenband, oder in Form einer textilen Flächenzubereitung vorliegen, beispielsweise als Vlies, Flor, Gewebe oder Gewirk.

In der einfachsten Ausführungsform umfaßt die erfindungsgemäße Zubereitung ausschließlich Alginate als nicht antiseptisch wirksames Material. Bei der erfindungsgemäßen Zubereitung kann es sich aber auch um eine Kombination von antiseptisch imprägnierten Alginatfasern mit zusätzlichen Materialien handeln, die für die Herstellung von Wundauflage geeignet sind. Geeignete Materialien für die Kombination mit Alginatfasern sind beispielsweise Materialien auf Kollagen-Basis, Cellulose und Cellulosederivate, insbesondere Carboxymethylcellulose, Pektine, sowie synthetischen Fasern und sogenannte Superabsorber, bei denen es sich vorzugsweise um Polyacrylate handelt. Diese Materialien können ebenfalls mit einem antiseptisch wirksamen Mittel versehen sein, bevor sie mit Alginatfasern zu den gewünschten Produkten weiterverarbeitet werden.

Bei diesen Ausführungsformen kann der Anteil an Alginatfasern, bezogen auf das Gewicht der nicht antiseptisch wirksamen Komponenten der Zubereitung, 5 bis 95 Gew.-% betragen. Vorzugsweise stellen die Alginatfasern mengenmäßig den größeren Anteil an nicht antiseptisch wirksamen Komponenten der Zubreitung dar. Der Anteil an Alginatfasern beträgt bei diesen Ausführungsformen 50 bis 95 Gew.-%, vorzugsweise 60 bis 90 Gew.-% und besonders bevorzugt 70 bis 80 Gew.-%. Demzufolge beträgt der Anteil der zusätzlichen nicht antiseptisch wirksamen Materialien 5 bis 50 Gew.-%, vorzugsweise 10 bis 40 Gew.-% und besonders bevorzugt 20 bis 30 Gew.-%.

Es sind aber auch Ausführungsformen möglich, bei denen der Anteil an Alginatfasern in der Kombination mit anderen Materialien 50 Gew.-% oder weniger beträgt, bezogen auf das Gewicht der nicht antiseptisch wirksamen Komponenten der Zubereitung. Bei diesen Ausführungsformen beträgt der Anteil an Alginatfasern 5 bis 50 Gew.-%, vorzugsweise 10 bis 40 Gew.-% und besonders bevorzugt 20 bis 30 Gew.-%, bezogen auf das Gewicht der nicht antiseptisch wirksamen Komponenten der Zubereitung. Bei diesen Ausführungsformen beträgt der Anteil an dem zusätzlichen, nicht antiseptisch wirksamen Material 50 bis 95 Gew.-%, vorzugsweise 60 bis 90 Gew.-% und besonders bevorzugt 70 bis 80 Gew.-%, ebenfalls bezogen auf das Gewicht der nicht antiseptisch wirksamen Komponenten der Zubereitung.

Die erfindungsgemäße Zubereitung kann auch in Form von Alginatlösungen oder Alginatgelen vorliegen. Vorzugsweise handelt es sich bei den Alginatgelen um eine Mischung aus Natrium-Alginat und Calcium-Alginat, so daß es die gewünschte Viskosität aufweist.

In einer besonders bevorzugten Ausführungsform liegt die erfindungsgemäße Zubereitung als Lyophilisat einer Alginatlösung vor.

Die erfindungsgemäße Zubereitung umfaßt mindestens eine wasserlösliche, antiseptisch wirksame Substanz aus der Gruppe, die Biguanid-Derivate, Octenidin und Taurolidin umfaßt. Geeignete antiseptisch wirksame Substanzen sind beispielsweise die Salze des Chlorhexidins, die Salze des Octenidins oder die Salze des Polyhexamethylenbiguanids sein. Beispiele für geeignete Salze sind Chlorhexidindihydrochlorid, Chlorhexidin-diacetat, Chlorhexidin-D-digluconat, Octenidin-dihydrochlorid, Octenidin-disaccharin und das besonders bevorzugte Polyhexamethylenbiguanid-hydrochlorid.

Die antiseptisch wirksame Substanz ist vorzugsweise in einer Menge von 0,1 bis 40 Gew.-%, vorzugsweise in einer Menge von 0,5 bis 10 Gew.-%, bezogen auf das Trockengewicht der Alginatzubereitung, in der Zubereitung enthalten.

Die erfindungsgemäßen Zubereitungen können durch direkte Imprägnierung der Alginatfasern mit dem Antiseptikum hergestellt werden, indem die Alginatfasern vor ihrer Verarbeitung zum gewünschten Produkt mit dem Antiseptikum imprägniert werden. Es ist jedoch auch möglich, das fertige Produkt, beispielsweise die Wundauflage, mit dem Antiseptikum zu imprägnieren.

Für das Imprägnierten können die Alginatfasern oder das aus bzw. mit Hilfe der Alginatfasern hergestellte Produkt, welches gegebenenfalls weitere Materialien umfaßt, durch Besprühen mit einer Antiseptikum-haltigen Lösung oder durch Eintauchen und/oder Umwälzen der Alginatfasern bzw. des Produktes in einer Antiseptikum-haltigen Lösung, so daß die mit dem Antiseptikum getränkten Alginatfasern bzw. Produkte nach Trocknung den gewünschten Gehalt an Antiseptikum aufweisen.

Es ist aber auch möglich, Alginatfasern während ihrer Herstellung mit einem Antiseptikum zu imprägnieren, indem das Antiseptikum bereits im Fällbad enthalten ist oder unmittelbar vor dem Trocknen der naßgesponnenen Alginatfasern auf diese getropft oder gesprüht wird.

Als Lösungsmittel für das Antiseptikum wird Wasser bevorzugt, aber andere, pharmakologisch verträgliche Lösungsmittel kommen ebenfalls in Betracht. Neben Pufferlösungen ist beispielsweise Ethanol geeignet sowie beliebige Mischungen der geeigneten Lösungsmittel.

### Beispiel 1

10 x 10 cm große Stücke (Gewicht: 1 g) einer Kompresse aus Calcium-Alginatfasern (Fa. SFM Limited) wurden in eine Schale gegeben und mit 20 ml einer wäßrigen PHMB-Lösung (Cosmocil QC, 10 mg PHMB absolut; Fa. Arch Chemicals) befeuchtet. Für eine 1%-ige Beladung der Kompresse mit Antiseptikum wurde ein Stück der Kompresse mit der 10-fachen Masse einer 0,1 %-igen PHMB-Lösung mit einer Pipette gleichmäßig über die gesamte Fläche befeuchtet und bei 65°C 20 min in der Schale getrocknet. Die befeuchteten Stücke wurden durch vollständiges Trocknen mit PHMB imprägniert, verpackt und anschließend durch Bestrahlung mit gammaStrahlen sterilisiert. Die weiteren im Rahmen dieser Untersuchungen hergestellten und verwendeten PHMB-Beladungen sind Tabelle 1 zu entnehmen.

Die antimikrobielle Wirkung der mit PHMB imprägnierten Wundauflagen wurde mittels Agar-Diffusions-Tests untersucht.

Zu diesem Zweck wurden drei klinische Isolate (*Staphylococcus aureus* (ATCC 6538P), *Pseudomonas aeruginosa* (ATCC 9027) und *Candida albicans* (ATCC 10231)) für 24 h bei 30 bis 35°C in einem nicht selektiven Flüssigmedium kultiviert und anschließend mit einer 1%-igen NaCl-Lösung, die 1% Pepton enthielt auf **1x10⁸** CFU/ml (colony forming units) bzw. 3,8x10⁷ CFU/ml (*C*. *albicans*) verdünnt. Jeweils 100 µl der Verdünnungen wurden auf CSA- bzw. SDA-Platten (CSA = Casein-Soja-Pepton-Agar; SDA = Sabouraud-Dextrose-Agar)ausgestrichen. Die Agar-Platten wurden 3 bis 5 Minuten getrocknet.

Test-Plättchen (Durchmesser 34 mm) aus den imprägnierten Wundauflagen wurden mit sterilen Pinzetten auf die jeweiligen Agar-Platten transferiert, die anschießend mit 400 µl einer 0,5%-igen NaCl-Lösung überschichtet wurden. Die Platten wurden für 24 h bei 30 bis 35 °C bzw. 48 h bei 20 bis 25°C (*C. albicans*) inkubiert, bevor der Bereich der Hemmung ermittelt wurde.

Der Bereich der Hemmung wurde quantifiziert, indem der Durchmesser des Test-Plättchens vom Durchmesser des klaren Hemmhofs abgezogen und der resultierende Wert durch zwei geteilt wurde. Das Ergebnis entspricht dem Abstand von Plättchenrand zum Rand des Hemmhofs und wird in mm angegeben. Die Ergebnisse sind in Tabelle 1 dargestellt.

**Tabelle 1: Antiseptische Wirkung von PHMB-beladenem Gewebe aus Calcium-Alginatfasern**

| PHMB-Beladung | Hemmung der Ausbreitung von | | |
|---|---|---|---|
| | *S. aureus* | *P. aeruginosa* | *C. albicans* |
| 0,5 % | 1 mm | 0 mm | 1-2 mm |
| 1,0 % | 2-4 mm | 0-0,5 mm | 1-2 mm |
| 1,5 % | 3-4 mm | 0-0,5 mm | 1-2 mm |
| 2,0 % | 3-4 mm | 0-0,5 mm | 1-3 mm |

### Beispiel 2

5 x 5 cm große Stücke einer Wundauflage aus Calcium-Alginatfasern (Suprasorb A, Fa. Lohmann und Rauscher) wurden mit der zehnfachen. Masse einer PMM-Lösung befeuchtet, die 0,1, 0,5 oder 1 % PHMB enthielt. Die befeuchteten Wundauflagen wurden bei -50°C tiefgefroren und anschließend lyophilisiert. Nach Lyophilisiation betrugen die Beladung der imprägnierten Wundauflagen mit PHMB 1, 5 bzw. 10 Gew.-%.

Die antimikrobielle Wirkung der mit PHMB imprägnierten Wundauflagen wurde mittels Agar-Diffusions-Tests untersucht, wie in Beispiel 1 beschrieben.

Die mit PHMB imprägnierten Wundauflagen aus Calcium-Alginatfasern zeigten bereits bei geringer PHMB-Beladung eine deutliche antiseptische Wirkung gegenüber den gramnegativen *S. aureus*. Die Hemmzone um die Stücke wurde mit zunehmender PHMB-Beladung der Wundauflage größer.

Bei P. aeruginosa (grampositiv) konnte eine antiseptische Wirkung nur bei höheren PHMB-Beladungen nachgewiesen werden. Die Hemmzone war bei allen Konzentrationen klein. Ein Verkeimen der Test-Plättchen wurde jedoch mit allen PHMB-Beladungen effizient verhindert.

Eine deutliche Hemmzone um die Test-Plättchen wurde auch bei den Versuchen mit *C. albicans* beobachtet.

Diese Ergebnisse zeigen, daß durch Beladung von Calcium-Algianten mit Polyhexanid Wundauflage hergestellt werden können, die eine antiseptische Wirkung haben. Damit widersprechen die vorliegenden Ergebnisse der allgemeinen Auffassung in Fachkreisen, daß bei Anwendung des kationischen Polyhexanids bereit geringe Spuren negativer Ladung, beispielsweise in Form von Alginat-, Acrylat-, Lactat- oder Iodidionen, ausreichen, um dessen antiseptische Wirkung schnell zu inaktivieren.

Durch Beladung von Alginatfasern oder Produkten auf Basis von Alginatfasern lassen sich Wundauflage oder Verbände herstellen, die eine antiseptische Wundbehandlung ermöglichen, ohne daß auf die bei Verwendung von Alginaten geschätzten Vorteile verzichtet werden muß.

## Patentansprüche

1. Antiseptische Zubereitung zur Herstellung einer Wundauflage, umfassend mindestens ein Alginate und mindestens eine antiseptisch wirksame Substanz, die aus der Gruppe ausgewählt ist, die polyhenamethylenbiguanid, Salze des Polyhaxamethylenbiguanids, Octenidin, Salze des Octenidins und Taurolidin umfaßt.

2. Zubereitung nach Anspruch 1, **dadurch gekennzeichnet, daß** das Alginat aus der Gruppe der Wasserlöslichen Alginate ausgewählt ist.

3. Zubereitung nach Anspruch 1 oder 2, **dadurch gekeznnzeichnet, daß** das Alginat aus der Gruppe ausgewählt ist, die Alkali-Alginate, vorzugsweise Natrium-Alginat, Magnesium-Alginat und Ammonium-Alginat umfaßt.

4. Zubereitung nach Anspruch 1, **dadurch gekennzeichnet, daß** das Alginat aus der Gruppe der wasserunlöslichen Alginate ausgewählt ist.

5. Zubereitung nach Anspruch 1 oder 4, **dadurch gekennzeichnet, daß** das Alginate aus der Gruppe ausgewählt ist, die Calcium-Alginate und Zink-Alginat umfaßt.

6. Zubereitung nach einem der vorangehenden Ansprüche, **dadurch gekennzeichnet, daß** die antiseptisch wirksame Substanz wasserlöslich ist.

7. Zubereitung nach Anspruch 1, **dadurch gekennzeichnet, daß** das Salz der antiseptisch wirksamen Substanz aus der Gruppe ausgewählt ist, die aus Polyhexamethylenbiguanid-hydrochlorid, Octenidin-dihydrochlorid und Octenidin-disaccharin besteht.

8. Zubereitung nach einem der vorangehenden Ansprüche, **dadurch gekennzeichnet, daß** die antiseptisch wirksame Substanz in einer Menge von 0,1 bis 40 Gew.-%, vorzugsweise von 0,5 bis 10 Gew.-%, bezogen auf das Trockengewicht der Alginatzubereitung, in der Zubereitung enthalten ist.

9. Zubereitung nach einem der vorangehenden Ansprüche, **dadurch gekennzeichnet, daß** die Zubereitung zusätzlich zu antiseptisch imprägnierten Algniatfasern Materialien umfaßt, die aus der Gruppe ausgewählt sind, die aus Materialien auf Kollagenbasis, Pektin, Cellulose und Cellulosederivaten, insbesondere Carboxymethylcellulose, synthetischen Fasern und/oder Superabsorbern, vorzugsweise Polyacrylaten, besteht.

10. Zubereitung nach Anspruch 9, **dadurch gekennzeichnet, daß** der Anteil an dem zusätzliche Material 5 bis 50 Gew.-%, vorzugsweise 10 bis 40 Gew.-% und besonders bevorzugt 20 bis 30 Gew.-%, bezogen auf das Gewicht der nicht antiseptisch wirksame Komponenten der Zubereitung, beträgt.

11. Zubereitung nach Anspruch 9, **dadurch gekennzeichnet, daß** der Anteil an dem zusätzlichen Material 50 bis 95 Gel.-%, vorzugsweise 60 bis 90 Gew.-% und besonders bevorzugt 70 bis 80 Gew,-%, bezogen auf das Gewicht der nicht antiseptisch wirksamen Komponenten der Zubereitung, beträgt.

12. Zubereitung nach einem der vorangehenden Ansprüche, **dadurch gekennzeichnet, daß** sie in Form von Fasern, vorzugsweise von losen Fasern, eines Faserverbundes, vorzugsweise eines lose Faserverbunds, einer textilen Flächenzubereztung, vorzugsweise als Gewebe, Gewirke, Flor oder vlies, einer Lösung, eines Gels oder eines Lyophilisats vorliegt.

13. Verfahren zur Herstellung einer antiseptischen Alginatzubereitung, **dadurch gekennzeichnet, daß**
- Alginatfasern mit mindestens einer antiseptisch wirksamen Substanz imprägniert werden, die aus der Gruppe ausgewählt ist, die Polyhexamethylenbiguanid, salze des Polyhexametnylenbiguanids, Octenidin, Salze des Octenidins und Taurolidin umfaßt, indem
- die Alginatfasern oder die aus den Alginatfasern hergestellten Produkte mit einer antiseptisch wirksamen Lösung besprüht, betropft oder getränkt; oder
- die Alginatfasern durch ein die antiseptisch wirksame Substanz(en) enthaltendes Fällungsbad gewonnen; und anschließend getrocknet werden, oder
- eine Alginat-Lösung mit mindestens einer antiseptisch wirksamen Substanz versetzt wird.

14. Verfahren nach Anspruch 13, **dadurch gekennzeichnet, daß** das Salz der antiseptisch wirksamen Substanz aus der Gruppe ausgewählt ist, die aus Polyhexamethylenbiguanid-hydrochlorid, Octenidin-dihydrochlorid und Octenidin-disaccharin besteht.

15. verfahren nach einem der Ansprüche 13 oder 14, **dadurch gekennzeichnet, daß** das Trocknen mittels Gefriertrocknung erfolgt.

16. Verwendung einer Zubereitung nach einem der Ansprüche 1 bis 12 zur Herstellung von Wundauflage und Verbandmaterial.

17. Antiseptische Wundauflage, umfassend eine oder basierend auf einer Zubereitung nach einem der Ansprüche 1 bis 12.

## Claims

1. Antiseptic preparation for the production of a wound dressing, comprising at least one alginate and at least one antiseptically active substance, said antiseptically active substance being selected from the group comprising polyhexamethylene biguanide, salts of polyhexamethylene biguanide, octenidine, salts of octenidine, and taurolidine.

2. Preparation according to claim 1, **characterised in that** the alginate is selected from the group of the water-soluble alginates.

3. Preparation according to claim 1 or 2, **characterised in that** the alginate is selected from the group comprising the alkali alginates, preferably sodium alginate, magnesium alginate and ammonium alginate.

4. Preparation according to claim 1, **characterised in that** the alginate is selected from the group of the water-insoluble alginates.

5. Preparation according to claim 1 or 4, **characterised in that** the alginate is selected from the group comprising calcium alginate and zinc alginate.

6. Preparation according to any one of the preceding claims, **characterised in that** the antiseptically active substance is water-soluble.

7. Preparation according to claim 1, **characterised in that** the salt of the antiseptically active substance is selected from the group consisting of polyhexamethylene biguanide hydrochloride, octenidine dihydrochloride and octenidine disaccharin.

8. Preparation according to any one of the preceding claims, **characterized in that** the antiseptically active substance is contained in said preparation in an amount of 0.1 to 40%-wt., preferably 0.5 to 10%-wt., relative to the dry weight of the alginate preparation.

9. Preparation according to any one of the preceding claims, **characterised in that** said preparation, in addition to antiseptically impregnated alginate fibres, comprises materials selected from the group consisting of collagen-based materials, pectin, cellulose and cellulose derivatives, especially carboxymethyl cellulose, synthetic fibres und/or superabsorbers, preferably polyacrylates.

10. Preparation according to claim 9, **characterised in that** the portion of additional material is 5 to 50%-wt., preferably 10 to 40%-wt., and particularly preferably 20 to 30%-wt., relative to the weight of the non-antiseptically active components of the preparation.

11. Preparation according to claim 9, **characterised in that** the portion of additional material is 50 to 95%-wt., preferably 60 to 90%-wt., and particularly preferably 70 to 80%-wt., relative to the weight of the non-antiseptically active components of the preparation.

12. Preparation according to any one of the preceding claims, **characterised in that** it is present in the form of fibres, preferably loose fibres, a fibre composite, preferably a loose fibre composite, a textile fabric, preferably as a woven, a knitted fabric, a web or a nonwoven, a solution, a gel or a lyophilisate.

13. Process according to an antiseptic alginate preparation, **characterised in that**
- alginate fibres are impregnated with at least one antiseptically active substance which is selected from the group comprising polyhexamethylene biguanide, salts of polyhexamethylene biguanide, octenidine, salts of octenidine and taurolidine, by
- spraying or soaking the alginate fibres, or the products made from the alginate fibres, with an antiseptically active solution, or dripping said solution on to said fibres or products; or
- obtaining the alginate fibres by means of a precipitation bath containing the antiseptically active substance(s);
and subsequent drying, or
- at least one antiseptically active substance is added to an alginate solution.

14. Process according to claim 13, **characterised in that** the salt of the antiseptically active substance is selected from the group consisting of polyhexamethylene biguanide hydrochloride, octenidine dihydrochloride and octenidine disaccharin.

15. Process according to any one of claims 13 or 14, **characterised in that** drying is effected by lyophilisation.

16. Use of a preparation according to any one of claims 1 to 12 for the production of wound dressings and bandage material.

17. Antiseptic wound dressing comprising, or based on, a preparation according to any one of claims 1 to 12.

## Revendications

1. Préparation antiseptique pour la fabrication d'un pansement, comprenant au moins un alginate et au moins une substance à activité antiseptique qui est choisie parmi le groupe qui comprend le polyhexaméthylènebiguanide, des sels du polyhexaméthylènebiguanide, l'octénidine, des sels de l'octénidine et la taurolidine.

2. Préparation selon la revendication 1, **caractérisée en ce que** l'alginate est choisi parmi le groupe des alginates solubles dans l'eau.

3. Préparation selon la revendication 1 ou 2, **caractérisée en ce que** l'alginate est choisi parmi le groupe qui comprend des alginates de métaux alcalins de préférence l'alginate de sodium, l'alginate de magnésium et l'alginate d'ammonium.

4. Préparation selon la revendication 1, **caractérisée en ce que** l'alginate est choisi parmi le groupe des alginates insolubles dans l'eau.

5. Préparation selon la revendication 1 ou 4, **caractérisée en ce que** l'alginate est choisi parmi le groupe qui comprend l'alginate de calcium et l'alginate de zinc.

6. Préparation selon l'une quelconque des revendications précédentes, **caractérisée en ce que** la substance à activité antiseptique est soluble dans l'eau.

7. Préparation selon la revendication 1, **caractérisée en ce que** le sel de la substance à activité antiseptique est choisi parmi le groupe qui est constitué par le chlorhydrate de polyhexaméthylènebiguanide, le dichlorhydrate d'octénidine et le disaccharinate d'octénidine.

8. Préparation selon l'une quelconque des revendications précédentes, **caractérisée en ce que** la substance à activité antiseptique est contenue dans la préparation en une quantité de 0,1 à 40 % en poids, de préférence de 0,5 à 10 % en poids, rapportés au poids à sec de la préparation à base d'alginates.

9. Préparation selon l'une quelconque des revendications précédentes, **caractérisée en ce que** la préparation comprend, en plus de fibres d'alginates imprégnées avec un agent antiseptique, des matières qui sont choisies parmi le groupe qui est constitué par des matières à base de collagène, de la pectine, de la cellulose et des dérivés de la cellulose, en particulier la carboxyméthylcellulose, des fibres synthétiques et/ou des superabsorbants, de préférence des polyacrylates.

10. Préparation selon la revendication 9, **caractérisée en ce que** la fraction de la matière supplémentaire s'élève de 5 à 50 % en poids, de préférence de 10 à 40 % en poids et de manière particulièrement préférée de 20 à 30 % en poids, rapportés au poids des composants de la préparation exempts d'activité antiseptique.

11. Préparation selon la revendication 9, **caractérisée en ce que** la fraction de la matière supplémentaire s'élève de 50 à 95 % en poids, de préférence de 60 à 90 % en poids et de manière particulièrement préférée de 70 à 80 % en poids, rapportés au poids des composants de la préparation exempts d'activité antiseptique.

12. Préparation selon l'une quelconque des revendications précédentes, **caractérisée en ce qu'**elle est présente sous la forme de fibres, de préférence sous la forme de fibres détachées, d'un composite de fibres, de préférence d'un composite de fibres détachées, d'un produit plat textile, de préférence sous la forme d'un tissu, d'un tricot, d'un duvet ou d'un non tissé, d'une solution, d'un gel ou d'un lyophilisat.

13. Procédé pour la fabrication d'une préparation antiseptique à base d'alginates, **caractérisé en ce que**
- on imprègne des fibres d'alginates avec au moins une substance à activité antiseptique qui est choisie parmi le groupe qui comprend le polyhexaméthylènebiguanide, des sels du polyhexaméthylènebiguanide, l'octénidine, des sels de l'octénidine et la taurolidine,
- on applique une solution à activité antiseptique sur les fibres d'alginates ou sur les produits fabriqués à partir des fibres d'alginates par aspersion, par égouttage ou par imprégnation ; ou bien
- on récupère les fibres d'alginates via un bain de précipitation contenant la ou les substance(s) à activité antiseptique, et ensuite on sèche ; ou bien
- on ajoute à la solution d'alginates au moins une substance à activité antiseptique.

14. Procédé selon la revendication 13, **caractérisé en ce que** le sel de la substance à activité antiseptique est choisi parmi le groupe qui est constitué par le chlorhydrate de polyhexaméthylènebiguanide, le dichlorhydrate d'octénidine et le disaccharinate d'octénidine.

15. Procédé selon la revendication 13 ou 14, **caractérisé en ce que** le séchage a lieu par lyophilisation.

16. Utilisation d'une préparation selon l'une quelconque des revendications 1 à 12, pour la fabrication de pansements et d'une matière pour bandage.

17. Pansement antiseptique, comprenant une préparation selon l'une quelconque des revendications 1 à 12 ou à base de ladite préparation.
